# FASCICULE DE BREVET EUROPEEN

(11) **EP 1 382 693 B1**
(45) Date de publication et mention de la délivrance du brevet: **08.03.2006**
(21) Numéro de dépôt: 03017827.1
(22) Date de dépôt: 05.09.2000
(51) Int. Cl.: C12Q 1/68

(54) **Méthodes et compositions pour la détection d'évènements pathologiques**
Verfahren und Zusammensetzungen für die Detektion pathologischer Ereignisse
Methods and compositions for the detection of pathologic events

(30) Priorité: 16.09.1999 FR 9911563
(43) Date de publication de la demande: 21.01.2004
(62) Demande divisionnaire de: 00962576.5
(73) Titulaire: Exonhit Therapeutics S.A., 75017 Paris (FR)
(72) Inventeur: Tocque, Bruno, 92400 Courbevoie (FR); Bracco, Laurent, 75014 Paris (FR); Schweighoffer, Fabien, 94300 Vincennes (FR)
(74) Mandataire: Becker, Philippe

(56) Documents cités:
- EP-A- 0 791 660
- WO-A-99/46403
- "EXONHIT THERAPEUTICS: Spécialiste de l'analyse qualitative des gènes" LA GAZETTE DU LABORATOIRE, [en ligne] vol. 31, octobre 1998 (1998-10), XP002261040 Extrait de l'Internet: <URL:http://www.gazettelabo.tm.fr/2002arch ives/prives/1998/31exonhit.htm> [extrait le 2003-11-04]
- BACHMANN M ET AL.: "Different La/SS-B mRNA isoforms are expressed in salivary gland tissue of patients with primary Sjören's syndrome" JOURNAL OF AUTOIMMUNITY, vol. 9, 1996, pages 757-766, XP002261041
- TRÖSTER H ET AL.: "One gene, two transcripts: Isolation of an alternative transcript encoding for the autoantigen La/SS-B from a cDNA library of a patient with primary Sjögrens' syndrome" JOURNAL OF EXPERIMENTAL MEDICINE, vol. 180, 1994, pages 2059-2067, XP000925381
- DIATCHENKO L ET AL: "SUPPRESSION SUBTRACTIVE HYBRIDIZATION: A METHOD FOR GENERATING DIFFERENTIALLY REGULATED OR TISSUE-SPECIFIC CDNA PROBES AND LIBRARIES" PROCEEDINGS OF THE NATIONAL ACADEMY OF SCIENCES OF USA,US,NATIONAL ACADEMY OF SCIENCE. WASHINGTON, vol. 93, 1 juin 1996 (1996-06-01), pages 6025-6030, XP002911922 ISSN: 0027-8424
- LISITSYN N ET AL: "CLONING THE DIFFERENCES BETWEEN TWO COMPLEX GENOMES" SCIENCE, AMERICAN ASSOCIATION FOR THE ADVANCEMENT OF SCIENCE,, US, vol. 259, no. 5097, 12 février 1993 (1993-02-12), pages 946-951, XP000196441 ISSN: 0036-8075
- SPILSBURY K ET AL.: "Isolation of a novel macrophage-specific gene by differential cDNA analysis" BLOOD, vol. 85, no. 6, 1995, pages 1620-1629, XP000925451
- REY I ET AL.: "A role for Grb2 in apoptosis ?" CELL DEATH AND DIFFERENTIATION, vol. 2, avril 1995 (1995-04), pages 105-111, XP008024173
- SCHWEIGHOFFER F ET AL.: "Qualitative gene profiling: a novel tool in genomics and in pharmacogenomics that deciphers messenger RNA isoforms diversity" PHARMACOGENOMICS, vol. 1, no. 2, 2000, pages 187-197, XP008024170

## Description

La présente invention concerne de nouvelles compositions et méthodes pour la détection d'événements pathologiques. Elle concerne plus particulièrement des compositions et méthodes de détection d'événements pathologiques à distance. L'invention concerne également des outils, kits et compositions pour la mise en oeuvre de telles méthodes, ainsi que leurs utilisations dans le domaine de la santé humaine ou animale, ou en recherche expérimentale par exemple.

Avec le vieillissement de la population dans les pays industrialisés, apparaissent de nouveaux besoins en matière de diagnostic. Les maladies telles que les cancers, les maladies neurodégénératives seraient mieux prises en charge pour le bénéfice des patients et de la société si l'on disposait de diagnostics prédictifs de l'apparition et de l'évolution de la pathologie.

L'expérience montre que plus le diagnostic est posé précocement plus les chances de contrôler l'évolution probable de la maladie sont grandes. C'est le cas, très clairement des cancers. Les campagnes de détection précoce des cancers du sein par mammographie systématique ont permis d'améliorer l'espérance de survie à ces cancers. De même, on peut supputer qu'une prise en compte précoce des patients développant une maladie d'Alzheimer permettrait de ralentir significativement son évolution.

Ces maladies, telles les cancers et maladies neurodégénératives, ont une incidence qui augmente fortement avec l'âge de la population. Il est probable que ces maladies prennent des années avant de s'installer et de pouvoir être détectées. Il ressort par exemple que l'accumulation d'altérations successives au niveau du génome humain est requise pour aboutir à l'initiation de cancers. De même des études génétiques réalisées dans des populations choisies présentant une incidence forte de maladie d'Alzheimer, associées à des expériences de génétique chez l'animal, soulignent également le caractère multifactoriel de l'initiation de cette pathologie.

Ces pathologies liées au vieillissement présentent des caractéristiques communes, telles que :
- des altérations cellulaires survenant à la suite de déséquilibres de l'environnement des tissus incriminés liés à des agressions physiques, chimiquesou biologiques ;
- la contribution des cellules du système immunitaire.

Si les altérations des tissus incriminés ne sont préférentiellement identifiées qu'à la suite de biopsies, il se pourrait que des altérations des cellules immunitaires, reflet d'un développement pathologique en cours, puissent être détectées à distance des foyers de développement de ces pathologies, puisque la plupart des cellules du système immunitaire évoluent entre les tissus et le compartiment sanguin ou les ganglions lymphatiques.

Les cellules lymphocytaires et les macrophages sont les principaux médiateurs de la réponse immunitaire cellulaire. Lymphocytes et macrophages sont présents dans les tissus ainsi que dans le sang. Ce sont ces cellules qui entrent les premiers en contact avec les tissus étrangers à l'organisme. Ces cellules macrophagiques dégradent les tissus et substances incriminés. Les peptides issus des protéines dégradées sont ensuite fixés par des molécules du système majeur d'histocompatibilité de classe II qui les amènent à la surface du macrophage où ces complexes sont reconnus par les lymphocytes T. D'autres systèmes de présentation de peptides et d'activation des réponses immunitaires existent et ont été décrits notamment dans le cas du développement de cancers.

Aujourd'hui les diagnostics de ce type de maladies sont faits alors que la pathologie est installée. Pour ce qui est des cancers, par exemple, le diagnostic est posé à partir d'une imagerie médicale et d'un diagnostic morphologique des tissus obtenus à la suite de biopsies. Pour une pathologie comme la maladie d'Alzheimer c'est un faisceau d'observations médicales qui permet de poser le diagnostic.

Il existe donc un réel besoin de disposer d'outils et de méthodes permettant de dépister de manière précoce, simple et fiable l'apparition de pathologies, notamment de pathologies liées à un dérèglement des mécanismes de régulation des voies de signalisation cellulaire, en particulier des pathologies caractérisées par une hyper-prolifération cellulaire (cancer, dégénérescence nerveuse, sténose, etc).

L'introduction des techniques issues de la biologie moléculaire, associée à la bio-informatique, a permis de concevoir des librairies (ou banques) de fragments d'ADN caractérisant un état pathologique donné, permettant de déterminer à partir d'un échantillon très petit d'un tissu quelconque, la présence ou l'absence de références pathologiques.

La présente invention décrit à présent une nouvelle approche pour la détection de pathologies in vitro. Plus particulièrement, la présente invention décrit de nouvelles méthodes et compositions pour la détection d'événements pathologiques, notamment de signatures génétiques pathologiques. L'invention décrit en outre des méthodes et compositions utilisables pour la détection à distance d'événements pathologiques, c'est-à-dire sur des matériels biologiques distincts des tissus pathologiques. Les compositions et méthodes de l'invention offrent à présent aux cliniciens, biologistes et industriels de nouvelles solutions pour le diagnostic *in vitro*, fondées sur des méthodes directes, rapides, sensibles et économiques, et automatisables.

Plus particulièrement, la présente invention repose notamment sur la mise en évidence qu'il est possible de déterminer, à partir d'échantillons biologiques comprenant des cellules circulantes, la présence ou te risque de développement d'une pathologie. Plus particulièrement, l'invention repose sur la mise en évidence qu'il est possible de détecter dans un échantillon biologique comprenant des cellules sanguines, l'existence d'une pathologie, y compris à des stades très précoces d'initiation ou de développement, auxquels tout autre diagnostic existant serait inefficace.

Un premier objet de l'invention réside plus particulièrement dans un procédé de détection in vitro de la présence d'une pathologie chez un sujet, comprenant le prélèvement d'un échantillon de cellules sanguines du sujet et la détermination de la présence, dans cet échantillon, de cellules sanguines présentant un état physiologique caractéristique de la pathologie.

Dans un mode particulier le procédé de l'invention comprend la détermination de la présence, dans l'échantillon, de cellules sanguines présentant une protéine ou un domaine protéique caractéristique de la pathologie. Dans ce contexte, le terme « présentant » désigne aussi bien la présence de cette protéine ou domaine protéique à l'intérieur de la cellule (dans n'importe quel compartiment cellulaire) que sa présence dans la membrane cellulaire ou à la surface de la cellule. Dans ce mode de réalisation, la présence de la protéine (ou du domaine protéique) peut être déterminée au moyen d'anticorps (ou de fragments ou dérivés d'anticorps), ou par toute autre technique connue de l'homme du métier.

Dans un autre mode particulier, le procédé de l'invention comprend la détermination de la présence, dans l'échantillon, de cellules sanguines présentant un profil génétique caractéristique de la pathologie.

Encore plus préférentiellement, le procédé de l'invention comprend la détermination de la présence, dans l'échantillon, de cellules sanguines présentant des altérations de l'expression génétique caractéristiques de la présence de la pathologie.

Ainsi, la présente invention repose d'une part sur l'utilisation de cellules sanguines pour la réalisation d'un test à distance de la présence d'un événement pathologique et, d'autre part, sur l'utilisation de technologies génomiques permettant de détecter des altérations de l'expression (en particulier de la transcription) du génome dans ces cellules.

Selon une variante particulière, la présente invention comprend donc plus préférentiellement la détermination de la présence, dans un échantillon biologique, de cellules sanguines présentant des altérations transcriptionnelles et/ou post-transcriptionnelles de l'expression génétique, caractéristiques de la présence d'une pathologie.

L'invention réside en la démonstration qu'il est possible de détecter une pathologie en cours de formation à partir de signatures génomiques identifiées dans les cellules sanguines, les foyers embryonnaires pathologiques pouvant exister dans des tissus nerveux comme le cerveau ou la moelle épinière (sites de maladies neurodégénératives) ou dans tout autre tissu par exemple à l'origine d'un cancer (sein, poumon, prostate, foie, tissu osseux etc.).

L'invention démontre d'une manière inattendue qu'il existe au niveau des cellules sanguines (préférentiellement des cellules nucléées, telles que lymphocytes, macrophages, monocytes, cellules dendritiques, etc.) des altérations transcriptionnelles et post-transcriptionnelles de l'expression génétique à la suite d'interaction(s) directe(s) ou indirecte(s) avec les cellules en cours d'initiation pathologique.

Plus particulièrement, l'invention démontre d'une manière inattendue qu'il existe au niveau des cellules sanguines (préférentiellement des cellules nucléées, lymphocytes, macrophages, monocytes, cellules dendritiques, etc.) des altérations de la transcription qualitatives des gènes à la suite d'interaction(s) directe(s) ou indirecte(s) avec les cellules en cours d'initiation pathologique.

Selon une autre variante préférée, le procédé de l'invention comprend (i) la préparation d'acides nucléiques de l'échantillon et (ii) l'hybridation des acides nucléiques préparés avec au moins une banque d'acides nucléiques caractéristiques d'un état pathologique, le profil d'hybridation indiquant la présence de cellules sanguines caractéristiques de la pathologie dans l'échantillon.

Plus particulièrement, dans un mode de mise en oeuvre de l'invention, la banque utilisée comprend des acides nucléiques spécifiques de gènes dont le niveau d'expression est modifié dans une cellule sanguine provenant d'un organisme présentant une situation pathologique.

Dans un autre mode de mise en oeuvre de l'invention, la banque utilisée comprend des acides nucléiques spécifiques de formes d'épissages de gènes, caractéristiques d'une cellule sanguine provenant d'un organisme présentant une situation pathologique.

L'invention est basée notamment sur une méthode originale, l'analyse qualitative des différences liées à la présence d'insertions ou de délétions (épissages alternatifs) dans des régions essentielles pour la fonction des produits des gènes. Ces insertions et délétions sont précisément régulées et sont caractéristiques des états physiologiques et physiopathologiques (notamment prolifératifs et différenciés) des cellules de l'organisme. Ce niveau de régulation est affecté au cours de l'initiation, du maintien et du développement d'un grand nombre de pathologies. Dans un mode préféré, l'invention réside donc aussi dans l'application d'une technologie génomique destinée à analyser systématiquement ces dérégulations à la mise au point de diagnostics prédictifs. L'invention permet ainsi d'identifier les gènes dérégulés dans des cellules circulantes lors d'événements pathologiques, et d'utiliser ces événements génétiques qualitatifs dans des tests diagnostiques, prédictifs ou de détection d'événements pathologiques, qui contribuent à la maîtrise globale des dépenses de santé.

Dans la perspective d'identifier des marqueurs d'expression génétique spécifiquement présents dans les cellules sanguines d'un organisme présentant une pathologie, par exemple à un stade trop précoce pour pouvoir être diagnostiqué par examens cliniques ou tests diagnostics classiques, la présente invention propose avantageusement d'identifier des altérations post-transcriptionnelles. En effet ces altérations sont principalement la conséquence de la modification de la régulation d'une étape-clef de l'expression génique : l'épissage. Les variations d'épissage modifient de façon qualitative les ARN en incluant ou en excluant de ceux-ci des exons ou introns dont la présence ou l'absence liée à une situation physiopathologique donnée peut fournir la base pour un diagnostic. Ce diagnostic peut être basé sur l'utilisation de PCR ou d'hybridations qui permettent de détecter spécifiquement la séquence épissée de façon différentielle entre les deux situations. Souvent les variations d'épissage, par utilisation d'exon(s) alternatif(s) ou par rétention d'intron(s) dans un ARN messager affectent la séquence de la protéine correspondante. Ces différences dans l'enchaînement d'acides aminés permettent d'envisager un diagnostic base sur l'utilisation d'anticorps qui reconnaissent spécifiquement la séquence protéique alternative.

Comme indiqué ci-avant, le procédé de l'invention repose notamment sur l'utilisation de cellules circulantes comme matériel biologique. Plus particulièrement, il s'agit de cellules sanguines, et de préférence de cellules nucléées. On peut citer notamment les lymphocytes, macrophages, monocytes, cellules dendritiques, etc. Ces cellules peuvent être prélevées chez un sujet par toute technique connue de l'homme du métier, cytaphérèse, gradients Ficoll, préparation de cellules mononucléées du sang périphérique, etc. Pour la mise en oeuvre de la présente invention, les différentes populations de cellules sanguines peuvent être séparées les unes des autres, pour n'utiliser qu'un type particulier, présentant une signature génomique spécifique. Toutefois, le test de l'invention peut également être réalisé sur un échantillon biologique comprenant de cellules sanguines non séparées. Par ailleurs, les cellules circulantes peuvent également être (ou comprendre) des cellules tumorales, détachées du tissu pathologique, par exemple dans le cas de processus de métastases. Les acides nucléiques peuvent être préparés de l'échantillon selon toute technique connue de l'homme du métier (lyse cellulaire, extraction, isolement des ARN, etc.). En outre, ces acides nucléiques sont préférentiellement traités préalablement à l'étape d'hybridation, par exemple pour produire de ADNc, pour amplifier ces acides nucléiques, pour les marquer, etc. A cet égard, le marquage peut être par exemple radioactif, enzymatique, fluorescent, colorimétrique, ou de toute autre nature. Typiquement, le procédé de l'invention comprend le prélèvement d'un échantillon biologique sanguin, le traitement des cellules sanguines pour libérer les acides nucléiques, l'amplification des acides nucléiques (et, le cas échéant, leur transcription inverse), le marquage des acides nucléiques et leur hybridation sur la ou les banques.

Le procédé de l'invention peut être utilisé pour détecter la présence d'une pathologie (ou d'un événement pathologique), c'est-à-dire l'existence de mécanismes cellulaires caractéristiques d'une situation d'initiation ou de développement d'une pathologie, même si les symptômes cliniques ne sont pas encore apparents. Le procédé de l'invention peut à cet égard permettre également la détection in vitro du stade d'évolution d'une pathologie chez un sujet. Ainsi, les signatures génétiques des cellules évoluent en fonction du stade d'avancement de la pathologie, et il est possible de détecter, grâce à des banques spécifiques, l'évolution d'une pathologie. D'autre part, le procédé de l'invention permet aussi de détecter in vitro la localisation d'une pathologie chez un sujet, c'est-à-dire par exemple l'origine tissulaire du foyer pathologique.

Comme indiqué ci-avant, le procédé de l'invention peut être mis en oeuvre pour détecter différents types de pathologies, notamment des pathologies associées à des dérégulations des voies de signalisations cellulaires. Il peut s'agir de pathologies liées au vieillissement, comme par exemple les maladies neurodégénératives, ou de toute autre pathologie impliquant notamment une prolifération cellulaire anormale (cancer, sténose, etc.).

Selon un mode particulier, l'invention concerne un procédé tel que défini ci-avant pour la détection in vitro de la présence, du stade d'évolution et/ou de la localisation d'une pathologie neurodégénérative.

Selon un autre mode particulier, l'invention concerne un procédé tel que défini ci-avant pour la détection in vitro de la présence, du stade d'évolution et/ou de la localisation d'une pathologie cancéreuse. Il peut s'agir de cancers variés, comme par exemple de tumeurs solides (hépatiques, pulmonaire, tête et cou, mélanome, foie, vessie, sein, etc.).

L'invention décrit également un procédé de détection in vitro de cellules sanguines caractéristiques d'un état pathologique, comprenant le prélèvement d'un échantillon de cellules sanguines chez un sujet et la détermination de la présence, dans cet échantillon, de cellules sanguines présentant un profil génétique caractéristique d'une pathologie.

Comme décrit ci-avant, l'invention repose en partie sur la constitution et l'utilisation de banques d'acides nucléiques caractéristiques d'un état pathologique. Dans un premier mode de réalisation, il s'agit de banques (ou de préparations) d'acides nucléiques, comprenant des acides nucléiques spécifiques de gènes dont le niveau d'expression est modifié dans une cellule sanguine provenant d'un organisme dans une situation pathologique.

Selon un autre mode de réalisation, il s'agit de banques (ou de préparations) d'acides nucléiques comprenant des acides nucléiques spécifiques de formes d'épissages de gènes, caractéristiques d'une cellule sanguine provenant d'un organisme dans une situation pathologique.

Les préparations et banques de l'invention peuvent être déposées sur des supports, affinées et mélangées, comme il sera décrit plus en détails dans la suite du texte.

L'invention décrit encore des méthodes pour la préparation de telles banques. En particulier, ces méthodes comprennent (i) l'obtention d'une première préparation d'acides nucléiques à partir d'une cellule sanguine isolée d'un organisme présentant une pathologie, (ii) l'obtention d'une préparation d'acides nucléiques de référence à partir d'une cellule sanguine isolée d'un organisme ne présentant pas ladite pathologie, (iii) une étape d'hybridation entre ladite première préparation et la préparation de référence, et la récupération des acides nucléiques caractéristiques de la cellule sanguine provenant de l'organisme en situation de pathologie.

L'invention décrit également des procédés de préparation de banques d'acides nucléiques caractéristiques du stade d'évolution d'une pathologie, comprenant (i) l'obtention d'une première préparation d'acides nucléiques à partir d'une cellule sanguine isolée d'un organisme présentant une pathologie à un stade d'évolution déterminé, (ii) l'obtention d'une préparation d'acides nucléiques de référence à partir d'une cellule sanguine isolée d'un organisme présentant ladite pathologie à un stade d'évolution différent, (iii) une étape d'hybridation entre ladite première préparation et la préparation de référence, et (iv) la récupération des acides nucléiques caractéristiques de la cellule sanguine provenant de l'organisme au stade d'évolution déterminé de la pathologie.

Comme il sera expliqué en détails plus loin, l'étape de récupération des clones peut comprendre soit la récupération des clones d'acides nucléiques non hybridés, soit la récupération, à partir des hybrides formés, de clones d'acides nucléiques spécifiques de formes d'épissages de gènes.

L'invention concerne également tout kit utilisable pour la mise en oeuvre d'un procédé tel que décrit ci-avant comprenant une banque d'acides nucléiques comprenant des acides nucléiques spécifiques d'altérations d'expression génétique caractéristiques de cellules sanguines d'un organisme en situation pathologique.

### Identification de marqueurs spécifiques des modifications transcriptionnelles et post-transcriptionnelles

Comme indiqué ci-avant, l'invention décrit des procédés de préparation de banques d'acides nucléiques caractéristiques du stade d'évolution d'une pathologie, comprenant (i) l'obtention d'une première préparation d'acides nucléiques à partir d'une cellule sanguine isolée d'un organisme présentant une pathologie à un stade d'évolution déterminé, (ii) l'obtention d'une préparation d'acides nucléiques de référence à partir d'une cellule sanguine isolée d'un organisme présentant ladite pathologie à un stade d'évolution différent, (iii) une étape d'hybridation entre ladite première préparation et la préparation de référence, et (iv) la récupération des acides nucléiques caractéristiques de la cellule sanguine provenant de l'organisme au stade d'évolution déterminé de la pathologie.

Les méthodes de l'invention comprennent plus particulièrement la constitution de clones et de banques d'acides nucléiques à partir d'ARN(s) extraits de différentes pathologies, à différents stades de leur progression, et obtenues aussi bien à partir de tissus pathologiques que des cellules sanguines dont l'expression génétique a été affectée par ces tissus. L'obtention de ces clones et de ces banques se fait avantageusement par des techniques d'analyses différentielles de l'expression génétique. Les signatures différentielles obtenues sont donc spécifiques de différences entre tissu malade et tissu sain d'une part et cellules sanguines de malade et cellules sanguines de témoin sain d'autre part. Ces signatures peuvent donc être exprimées préférentiellement soit dans les échantillons pathologiques soit dans les échantillons contrôles.

Les populations d'acides nucléiques servant à l'obtention de clones ou à la constitution de banques sont par exemple des ARN (totaux ou messagers) des cellules extraits d'une situation pathologique et des ARN (totaux ou messagers) correspondant à une situation contrôle, ou des acides nucléiques dérivés des ces ARN totaux ou messagers (par transcription inverse, amplification, clonage dans des vecteurs, etc.). Ces acides nucléiques peuvent être préparés selon les techniques connues de l'homme du métier. Brièvement, ces techniques comprennent généralement une lyse des cellules, tissu ou échantillon, et l'isolement des ARNs par des techniques d'extraction. Il peut s'agir en particulier d'un traitement au moyen d'agents chaotropiques tels que le thiocyanate de guanidium (qui détruit les cellules et protège les ARN) suivi d'une extraction des ARN au moyen de solvants (phénol, chloroforme par exemple). De telles méthodes sont bien connues de l'homme du métier (voir Maniatis et al., Chomczynski et al., Anal. Biochem. 162 (1987) 156), et peuvent être aisément pratiquées en utilisant des kits disponibles dans le commerce tels que par exemple le kit US73750 (Amersham) pour les ARN totaux. Il n'est pas nécessaire que les ARN utilisés soient parfaitement purs, et notamment il n'est pas gênant que des traces d'ADN génomique ou d'autres composants cellulaires (protéine, etc.) subsistent dans les préparations, dès lors qu'ils n'affectent pas significativement la stabilité des ARNs. En outre, de manière facultative, il est possible d'utiliser non pas des préparations d'ARN totaux mais des préparations d'ARN messagers. Ceux-ci peuvent être isolés, soit directement à partir de l'échantillon biologique soit à partir des ARN totaux, au moyen de séquences polyT, selon les méthodes classiques. L'obtention d'ARN messagers peut à cet égard être réalisée au moyen de kits commerciaux tels que par exemple le kit US72700 (Amersham). Les ARN peuvent également être obtenus directement à partir de banques ou autres échantillons préparés à l'avance et/ ou accessibles dans des collections, conservés dans des conditions appropriées.

L'hybridation peut être réalisée dans différentes conditions, qui peuvent être ajustées par l'homme du métier. De préférence, on utilise pour l'hybridation un excès de la population d'acides nucléiques dérivés de la situation de dérégulation par rapport à la population d'acides nucléiques dérivés de la situation de contrôle.

A partir du produit de la réaction d'hybridation, deux types principaux d'approches peuvent être utilisés pour isoler les clones caractéristiques de dérégulations (pathologiques) selon l'invention. La première, purement quantitative, permet de générer une préparation d'acides nucléiques regroupant l'ensemble (ou une part significative) des clones résultant d'une différence de niveau d'expression entre les deux situations. De tels clones (et banques) sont obtenus selon les techniques connues d'hybridation soustractive, consistant essentiellement à éliminer les hybrides formés lors de l'étape d'hybridation, pour ne conserver que les clones non hybridés, caractéristiques de la situation de dérégulation par rapport à la situation de contrôle choisie.

Dans un mode préféré de mise en oeuvre, on utilise cependant un procédé qualitatif, qui permet de générer une préparation d'acides nucléiques regroupant l'ensemble (ou une partie importante) des clones résultant d'altérations génétiques fonctionnelles caractéristiques de la situation de dérégulation par rapport à la situation de contrôle choisie. Plus particulièrement, une telle banque qualitative comprend non pas l'ensemble des clones dont l'expression est modifiée, mais par exemple les clones correspondant à des épissages ou à des délétions différentielles entre les deux situations. Compte tenu du rôle des épissages alternatifs dans les voles de régulation et de transformation cellulaires, de telles, préparations (et banques) comportent avantageusement des clones ayant une valeur fonctionnelle importante, et donc susceptibles de refléter des modifications génétiques impliquées dans la situation de dérégulation. De tels clones permettent donc de constituer des banques plus prédictives et de générer des marqueurs génétiques plus représentatifs.
La constitution de telles banques qualitatives peut être réalisée par isolement à partir des hybrides formés lors de l'étape d'hybridation, des régions d'acides nucléiques correspondant à des épissages différentiels ou à des délétions. Selon les méthodes employées, ces régions correspondent soit aux régions non appariées, soit aux régions appariées.

Ces deux approches sont décrites plus en détails dans ce qui suit.

### Production et utilisation de banques différentielles quantitatives

Dans un premier mode de mise en oeuvre, on utilise donc dans la présente invention une banque différentielle quantitative, c'est à dire une banque comprenant des clones d'acides nucléiques correspondant à des gènes dont le niveau d'expression est modifié dans des cellules en situation pathologique par rapport à une situation contrôle. De telles banques peuvent être issues par exemple d'analyses différentielles quantitatives, et regrouper les séquences dont l'expression est augmentée ou diminuée lors de phénomènes de dérégulation cellulaire. Les méthodologies pour établir ce type de banque sont connues de l'homme de métier et peuvent être regroupées dans les catégories suivantes :

### Soustraction électronique à partir de Séquençage à Haut Flux

Ce procédé est basé sur le séquençage aléatoire d'un certain nombre de cDNAs. Un moteur de recherche informatique peut ensuite être utilisé pour effectuer une soustraction entre deux situations analysées.

### « Serial Analysis of Gene Expression (SAGE) »

Ce procédé est basé sur la reconnaissance d'une signature associée à chaque cDNA en utilisant des enzymes de restriction et des oligonucléotides adaptateurs. Cette étiquette correspond à une partie de la séquence du cDNA (longue de 10 nucléotides afin d'identifier de façon non ambiguë le cDNA correspondant). Ces étiquettes sont ensuite assemblées pour être séquencées puis analysées (Velculescu et coll., Science, 1995, 270:484-487). Cette approche représente donc un raccourci vis-à-vis du séquençage systématique.

### « Nucleic Acid Arrays »

Cette méthode repose sur le dépôt d'acides nucléiques (oligonucléotides, fragments PCR, cDNAs) sur supports solides (membranes, plaques de verre, bio-puces) à plus ou moins haute densité. Des sondes provenant d'ARN messagers d'échantillons sains ou pathologiques sont ensuite utilisées pour hybridation afin d' identifier les messagers qui sont ou bien sur-exprimés ou bien réprimés.

### « Differential Display »

Cette technique utilise une amorce oligo-dT et des amorces aléatoires pour réaliser des réactions PCR sur des populations d' ADNc. Les produits de PCR sont alors comparés sur des gels très résolutifs. Les fragments exprimés de façon différentielle sont ensuite isolés et leur présence est confirmée par Northem-blots avant séquençage.
Plusieurs variantes de cette technologie ont été développées (Prashar et Weissman, PNAS, 1996, 93:659-663). Ces variantes diffèrent par leur amorces et par le choix des enzymes de restriction et des adaptateurs utilisés.
Comme la technologie SAGE, elles s'adressent aux extrémités 3' des cDNAs. Plusieurs kits sont également disponibles sur le marché afin de rendre accessible cette approche.

### Clonage par soustraction

Cette technique est basée sur l'élimination de cDNAs communs à deux échantillons que l'on désire comparer. Ainsi, différents kits de soustraction dans lesquels le cDNA 'tester' est hybridé à un excès de cDNA 'driver' sont proposés (Clontech). Le produit final est constitué d'un pool de fragments amplifiés par PCR dérivé des cDNAs exprimés de façon différentielle, qui peut être cloné dans un vecteur approprié pour analyse ultérieure. La technologie RDA (Representational Différence Analysis) est également basée sur ce principe de soustraction (Lisitsyn et coll., Science. 1993, 259 :946-951).

La mise en oeuvre de ces techniques d'analyses différentielles permet donc de générer des clones et des banques quantitatifs, c'est-à-dire regroupant l'ensemble des séquences dont l'expression est augmentée ou diminuée lors de phénomènes de dérégulation(s) cellulaire(s) impliqués dans des pathologiques.

### Production et utilisation de banques qualitatives différentielles

Dans un autre mode de mise en oeuvre, on utilise avantageusement dans la présente invention une banque différentielle qualitative, c'est à dire une banque comprenant des clones d'acides nucléiques dont une partie au moins de la séquence correspond à la séquence de gènes épissés différentiellement entre des cellules correspondant à une situation pathologique et une situation contrôle. Ce type de banque regroupe donc les séquences épissées de manière différentielle lors de processus de dérégulation pathologique.

L'utilisation de ce type de banque est particulièrement avantageuse. En effet, les différentes voies de signalisation qui sont altérées dans nombre de pathologies, comme les cancers et les maladies neurodégénératives par exemple, impliquent des gènes et donc des ARNm dont l'expression est régulée par épissage alternatif. De plus, un nombre croissant d'exemples fournis par la littérature montre que des formes d'ARN spécifiquement observées en pathologie sont le résultat d'une altération de l'épissage.
En relation avec l'originalité de l'invention, il faut souligner aussi que l'état d'activation des différents types cellulaires qui participent à la réponse immunitaire est régulé par des cascades de signalisation dont les acteurs sont régulés par épissage.

Ainsi, la démence Alzheimer, la maladie de Huntington, la maladie de Parkinson sont autant d'exemples de maladies ayant un réel impact économique et présentant une composante neurodégénérative. Même si la description des symptômes cliniques et l'identification de quelques gènes de susceptibilité ont permis de faire des pas significatifs dans la connaissance de ces maladies, les bases moléculaires soutenant le développement de ces maladies sont toujours très obscures. L'élucidation des cascades de signalisation, dérégulées dans ces états pathologiques, conduira d'une manière indubitable à la découverte de cibles propices à l'intervention diagnostique et thérapeutique. La littérature souligne l'importance des altérations des processus d'épissage des ARN.
- L'Amyotrophie Spinale est l'une des maladies génétiques les plus fréquentes. Deux gènes SMN1 et SMN2 codent pour des protéines identiques, la perte des deux allèles SMN1 et une altération de l'épissage du gène SMN2 concourent au développement de la maladie (Lorson et coll. Proc. Natl. Acad. Sci. USA 1999, 96 : 6307-6311).
- Dans des biopsies de patients atteints de démence Alzheimer , il a été détecté des altérations spécifiques de l'épissage du gène de présinilline PS1 (Isoe-Wada et coll. Eur. J. Neurol., 1999 : 163-167)
- Le transporteur au glutamate est d'une importance majeure dans des maladies neurodégénératives comme la Sclérose Amyotrophie Latérale ou l'épilepsie, par exemple. Des altérations de l'épissage de ce transporteur en affectent la fonctionnalité (Meyer et coll. Neurosci. Lett, 1998. 241 : 68-70).

De nombreux exemples d'inactivation d'activité anti-oncogène résultant d'épissages alternatifs des messagers correspondant sont aujourd'hui connus :
- Dans les carcinomes pulmonaires à petites cellules, le gène de la protéine p130 qui appartient à la famille RB (protéine du rétinoblastome) est muté au niveau d'un site consensus d'épissage. La conséquence de cette mutation est l'élimination de l'exon 2 qui a pour résultat une absence de synthèse de la protéine en raison de la présence d'un codon « stop » précoce. Cette observation a été la première à souligner l'importance des membres de la famille RB dans la tumorigenèse.
- Dans les cancers de la tête et du cou, l'un des mécanismes d'inactivation de p53 implique une mutation dans un site consensus d'épissage.
- Dans d'autres types de cancers du poumon, le gène de la protéine p16/INK4A. protéine qui est un inhibiteur des kinases cyclines-dépendantes CDK4 et CDK6 est muté au niveau d'un site donneur d'épissage. Le résultat de cette mutation est la production d'une protéine tronquée à demi-vie courte. Or la protéine p16 s'associe normalement à CDK4 et CDK6, empêchant leur association aux cyclines de type D et la phosphorylation en particulier de RB, ce qui a pour conséquence l'accumulation des formes hyphosphorylées, actives, de RB. En l'absence de p16, RB est inactivée par phosphorylation. Il est à noter que le locus p16 est en fait particulièrement complexe et qu'outre l'expression de p16, il permet celle de p19 par épissage alternatif. La protéine p19 qui n'a aucun acide aminé commun avec la protéine p16, peut s'associer au proto-oncogène MDM2 et bloquer le cycle cellulaire en présence de p53, exerçant ainsi une fonction « suppresseur de tumeur ».
- WT1, anti-oncogène qui code pour un répresseur transcriptionnel dont les altérations sont à l'origine des tumeurs de Wilms, est transcrit en plusieurs ARN messagers engendrés par épissages alternatifs. Dans les cancers du sein, les proportions relatives des différents variants sont modifiées par rapport au tissu sain, fournissant des outils diagnostics et des pistes pour comprendre l'importance des différents domaines fonctionnels de WT1 dans la progression tumorale.
- Ce même phénomène de modification des rapports entre différentes formes d'ARN messagers et d'isoformes protéiques est retrouvé pour la neurofibrine NF1 dans les neurofibromes.
- Cette notion de modulation des phénomènes d'épissage signant la progression tumorale est également renforcée par l'exemple de HDM2. Cinq épissages alternatifs de HDM2 sont en effet détectés dans les carcinomes ovariens et pancréatiques et, fait particulièrement intéressant, leur expression augmente selon le stade d'avancement tumoral.
- La LTBP, composant de la matrice extracellulaire de divers tissus intervenant dans la sécrétion et le stockage du TGF-β est aussi produite sous différentes isoformes. L'une d'elle, probablement moins sensible à la protéolyse, semble moduler l'activité biologique du TGF-β et pourrait être impliquée dans différents états physiopathologiques hépatiques.

Les réponses immunitaires cellulaires et humorales sont sous contrôle transcriptionnel. La littérature fournit des exemples nombreux d'isoformes natives produites par épissage alternatif impliquées dans ces réponses immunitaires.
- les récepteurs « éboueurs » des macrophages sont des glycoprotéines membranaires essentiels aux réponses physiologiques et pathologiques de ces cellules sanguines et leurs fonctions sont régulées par des isoformes générées par épissage (Gough et coll. J.Lipid Res ; 1998 : 39 :531-543.)
- L' activation des lymphocytes T requiert la présence fonctionnelle de plusieurs récepteurs et protéines régulatrices. Boriello et Coll. (J. Immunol. 1995: 155 :5490-5497) ont rapporté la présence d'isoformes du co-facteur d'activation B7, à la suite d'épissage alternatif de ce gène, soulignant ainsi la grande plasticité de la réponse immunitaire apportée par ces variants d'épissage.

Pour tenir compte de ces phénomènes et de cette complexité, et isoler ainsi des signatures spécifiques d'un état pathologique et présentes dans les cellules sanguines, le procédé de l'invention utilise avantageusement, comme marqueurs génétiques, des événements d'épissages caractéristiques de situations de dérégulation.

Pour ce faire, la présente invention utilise par exemple des banques d'acides nucléiques qualitatives différentielles produites selon la méthodologie « DATAS » décrite dans la demande de brevet internationale WO 99/46403. En particulier, de telles banques peuvent être préparées par hybridation entre la population d'acides nucléiques dérivée des cellules isolées de la circulation sanguine à partir de la situation pathologique et la population d'acides nucléiques dérivée des cellules circulantes de la situation de contrôle, et isolement, à partir des hybrides formés, des acides nucléiques correspondant à des épissages différentiels.

Dans cette approche, l'hybridation est préférentiellement réalisée en phase liquide. En outre, elle peut être effectuée dans tout dispositif approprié, tel que par exemple des tubes (Eppendorf, par exemple), des plaques, ou tout autre support adapté et couramment utilisé en Biologie Moléculaire. L'hybridation est avantageusement réalisée dans des volumes compris entre 10 et 1000 µl, par exemple entre 10 et 500 µl. Il est entendu que le dispositif utilisé et les volumes utilisés peuvent être aisément adaptés par l'homme du métier. Les quantités d'acides nucléiques utilisées pour l'hybridation sont également connues de l'homme du métier. En général, il est suffisant d'utiliser des microgrammes d'acides nucléiques, par exemple de l'ordre de 0,1 à 100 µg. Par ailleurs, il est possible d'utiliser les acides nucléiques dans un rapport driver/tester variant de 50 à 0,02 environ, de préférence de 40 à 0,1. De manière plus particulièrement avantageuse, on préfère que ce rapport soit proche ou supérieur à 1, avantageusement entre 1 environ et 10 environ. Il est bien entendu que ce rapport peut être adapté par l'homme du métier selon les conditions du procédé (quantités d'acides nucléiques disponibles, situations physiologiques, but poursuivi, etc.). Les autres paramètres de l'hybridation (temps, température, force ionique) sont également adaptables par l'homme du métier. De manière générale après dénaturation des "tester" et "driver" (par chauffage par exemple), l'hybridation est réalisée pendant environ 2 à 24 heures, à une température de 37°C environ (éventuellement soumise à des sauts de température), et dans des conditions standard de force ionique (pouvant varier de 0,1 à 5M NaCl par exemple). Il est connu que la force ionique est un des facteurs déterminant la stringence d'une hybridation, notamment dans le cas d'hybridation sur support solide.
Selon un mode de mise en oeuvre particulier de l'invention, l'hybridation est réalisée en émulsion phénolique, par exemple selon la technique PERT ("Phenol Emulsion DNA Reassociation Technique) décrite par Kohne D.E. et al. (Biochemistry, Vol. 16, N° 24, pp 5329-5341, 1977). Avantageusement, l'hybridation est réalisée en émulsion phénolique maintenue par thermocycles (sauts de température de 37°C environ à 60/65°C environ) et non par agitation, selon la technique décrite par Miller et Riblet (NAR 23 (1995) 2339).

Toute autre technique d'hybridation en phase liquide, de préférence en émulsion, peut être utilisée dans le cadre de la présente invention. Par ailleurs, l'hybridation peut également se faire avec l'un des partenaires immobilisé sur un support. Avantageusement, c'est le driver qui est immobilisé. Cela peut être réalisé notamment grâce à des amorces biotinylées ou par toute autre technique d'immobilisation connue de l'homme du métier.

A partir des populations d'acides nucléiques générées par hybridation, les marqueurs génétiques de l'invention (les clones caractéristiques des altérations génomiques qualitatives) peuvent être identifiés par toute technique connue de l'homme du métier. Dans le cas des hétéroduplex ARN/ADN, ces régions se présentent essentiellement sous forme de régions d'ARN non-appariées (boucles d'ARN), et peuvent être identifiées et clonées par séparation des hétéroduplex et des acides nucléiques simple-brin (excès d'acide nucléique n'ayant pas réagi), digestion sélective des ARN double-brins (domaines engagés dans les hétéroduplex), puis séparation des ARN simple-brin résultant et des ADN simple-brins. Dans le cas des hétérotriplex, ces régions d'épissages différentiels se présentent essentiellement sous forme de régions d'ADN double-brin et peuvent être identifiées et clonées par traitement en présence d'enzymes appropriées telles qu'une enzyme permettant de digérer les ARN, puis une enzyme permettant de digérer les ADN simple-brin. Les acides nucléiques ainsi obtenus sont directement sous forme d'ADN double-brin et peut être clonés dans tout vecteur approprié.

Il est entendu que d'autres variantes et conditions précises pour l'isolement des acides nucléiques, les hybridations et l'obtention des clones qualitatifs sont indiquées dans la demande WO 99/46043.

Ces méthodologies permettent de générer des clones et des banques d'acides nucléiques correspondant à des marqueurs génétiques qualitatifs qui permettent de distinguer les cellules sanguines d'une situation saine de celles d'une situation pathologique. Comme indiqué dans la section expérimentale, ces préparations d'acides nucléiques représentent des marqueurs particulièrement utiles pour diagnostiquer à partir d'un prélèvement sanguin des maladies neurodégénératives et des cancers.

### Diversité des banques

Les méthodologies décrites ci avant permettent donc de générer des ensembles de clones d'acides nucléiques caractéristiques des différences entre situations saine et pathologique. Chaque technique de préparation génère de nombreux clones, constituant des banques. Ces banques peuvent être mises en oeuvre telles quelles, déposées sur des supports ou modifiées par addition ou suppression de clones, regroupement de différentes banques, ajout de clones témoins, etc.

Les banques de l'invention peuvent comprendre par exemple de 10 à 50 000 clones, plus généralement de 10 à 10 000 clones, encore plus préférentiellement de 50 à 5000 clones. Les clones sont généralement déposés, de façon ordonnée, sur un ou plusieurs supports, de façon à faciliter l'analyse des résultats d'hybridation. Le support peut être en verre, nylon, plastique, fibre, etc., de manière générale tout support solide adapté à l'étalement d'acides nucléiques. Le dépôt des banques sur les supports peut être réalisé par des techniques conventionnelles connues de l'homme du métier, qui sont décrites par exemples dans la demande internationale WO 99/46403.

Les banques utilisées peuvent comprendre à la fois des clones d'acides nucléiques correspondant à des gènes dont le niveau d'expression est modifié (marqueurs génétiques quantitatifs) et des clones d'acides nucléiques dont une partie au moins de la séquence correspond à des exons ou à des introns épissés de façon différentielle entre une situation saine et une situation pathologique (marqueurs génétiques qualitatifs). Ainsi, les marqueurs génétiques peuvent être générés selon des approches différentes, puis mélangés pour obtenir une réponse aussi prédictive que possible.
Il est également possible de réunir au sein d'une même banque, sur un même support, des marqueurs de l'expression génétique exprimés spécifiquement dans les cellules présentes dans la circulation sanguine lors de pathologies différentes. L'hybridation d'une telle banque permet donc un suivi du développement de plusieurs pathologies à partir d'un même prélèvement sanguin.
Un objet de la présente invention réside donc également dans une préparation d'acides nucléiques comprenant des marqueurs génétiques qualitatifs et quantitatifs caractéristiques de dérégulation(s) cellulaire(s) présentes dans les cellules de la circulation sanguine et symptomatiques de pathologies. Un objet particulier réside dans une banque comprenant des marqueurs génétiques caractéristiques de différentes situations de dérégulation. L'invention a aussi pour objet tout support solide sur lequel au moins deux banques d'acides nucléiques caractéristiques de deux situations de dérégulation ont été déposées. A cet égard, l'invention concerne encore un procédé de préparation d'une puce à ADN utilisable pour le diagnostic de pathologies, comprenant le dépôt, sur un support solide, d'une ou plusieurs préparations d'acides nucléiques caractéristiques de situation(s) de dérégulation.

Par ailleurs, selon un mode préféré de mise en oeuvre, on utilise des banques d'acides nucléiques affinées par sélection des clones au fur et à mesure de l'utilisation, en fonction de leur implication effective dans différentes pathologies ou différents stades d'une même pathologie. Les banques initiales peuvent en effet comprendre par exemple l'ensemble des clones caractéristiques des événements génétiques d'une situation de dérégulation consécutive à l'établissement d'une pathologie. Puis, la mise en oeuvre du procédé de diagnostic de l'invention permet d'observer que certains des clones hybrident avec des sondes issues de stades précis et intermédiaires du développement de la pathologie. Ces clones peuvent donc être identifiés comme révélateurs de stades précoces et peuvent fournir un outil diagnostic très puissant capable d'anticiper tout autre critère d'examen clinique, tout autre outil diagnostic.

De manière plus spécifique, la présente invention décrit à présent l'identification et la caractérisation de tels clones, utilisables comme marqueurs génétiques de la présence et de l'évolution de pathologies.

L'une des applications majeures de l'identification et du clonage de ces marqueurs génétiques concerne l'évaluation du potentiel hybridant des ARN extraits de cellules sanguines d'un individu donné. Cette évaluation peut s'effectuer en hybridant une sonde représentant les ARN messagers des cellules de cet individu avec une ou plusieurs banques de signatures caractéristiques de situation(s) patholgique(s), telles que décrites ci avant. Cette application est décrite plus en détails dans ce qui suit.

### Méthodes d'analyse et de diagnostic des signatures de pathologies

L'invention permet de déterminer la présence de signatures spécifiques de différents stades de pathologies par hybridation d'un échantillon d'acides nucléiques de cellules présentes dans la circulation sanguine avec les marqueurs génétiques définis ci-dessus, le profil d'hybridation observé indiquant les dérégulations physiopathologiques de l'organisme dont est issu l'échantillon sanguin. Dans ce but, les marqueurs génétiques utilisés sont préférentiellement regroupés sous forme de banques, afin de fournir une réponse aussi prédictive que possible. L'un des avantages de la présente invention réside également dans le nombre important de marqueurs utilisés, qui rend l'information générée d'autant plus prédictive. Le caractère prédictif de l'information est en outre consolidé par la nature des marqueurs utilisés et préparés.

Un objet particulier de l'invention réside dans une méthode d'analyse du statut des cellules sanguines, comprenant au moins une étape d'hybridation entre a) un échantillon d'acides nucléiques de cellules sanguines et b) une banque d'acides nucléiques correspondant à des événements génétiques caractéristiques de situation(s) de dérégulation(s) de voie(s) de signalisation cellulaire, le profil d'hybridation indiquant les dérégulations physiopathologiques de l'organisme.

D'autres aspects et avantages de la présente invention apparaîtront à la lecture de la section expérimentale suivante, qui doit être considérée comme illustrative et non limitative.

### SECTION EXPERIMENTALE

### Exemple de maladies neurodégénératives : ALS

Les modèles animaux donnent accès à des échantillons biologiques qui permettent d'analyser différentes étapes du développement d'une pathologie et de comparer ces étapes à des témoins sains.
La sclérose amyotrophique latérale (SAL/ALS) est une maladie neurodégénérative, associée à différents types d'inclusions tels les corps de Lewis et caractérisée par une apoptose des motoneurones spinaux et corticaux dont l'issue fatale est parfois associée à une démence frontale. Des formes sporadiques, sans aucune mutation décrite, coexistent avec des formes familiales (FALS) associées à des mutations dans le gène SOD1 codant pour la superoxyde dismutase. Des souris transgéniques qui expriment le gène humain SOD1 portant l'une des mutations qui prévaut dans les FALS (mutation G93A) sont disponibles auprès de Jackson Laboratory, sous condition de prise d'une licence d'utilisation auprès de la NorthWestern University. L'apparition des symptômes d'ALS liés à la mutation G93A dans SOD1 n'est pas la conséquence d'une réduction de l'activité superoxyde dismutase mais d'un gain de fonction qui augmente la capacité de l'enzyme à générer des radicaux libres. Ce modèle reproduit en 120 jours l'issue fatale de la maladie avec des symptômes comparables à ceux de la maladie humaine. Ce modèle permet d'avoir accès à des échantillons cérébraux, spinaux et du sang périphérique.

### Identification de formes d'épissage spécifique du modèle d'ALS :

La société ExonHit Therapeutics développe une approche originale de criblage différentiel qualitatif basée sur la technologie DATAS (Differential Analysis of Transcripts Alternatively Spliced). Cette technologie fait l'objet d'une demande de brevet en Europe et aux Etats-Unis. Les séquences identifiées par DATAS peuvent dériver d'exons alternatifs ou de rétention d'introns dans une des deux situations physiopathologiques comparées. Les données obtenues caractérisent donc des modifications de l'expression de séquences d'ARN qui affectent des domaines fonctionnels de protéines. L'analyse qualitative différentielle est effectuée sur des échantillons d'animaux transgéniques et des contrôles syngéniques âgés de 60 et 100 jours. 60 jours correspondent à un stade qui précède de peu les premiers symptômes, mais qui est déjà caractérisé au niveau cérébral par des changements dans la physiologie cellulaire, dont notamment une altération du métabolisme mitochondrial. A 100 jours, 50% des motoneurones corticaux et spinaux sont morts et un processus actif d'apoptose neuronale est engagé parallèlement à une activation astrocytaire.
L'analyse qualitative différentielle est donc effectuée :
- à partir d'ARN extraits d'échantittons de cerveau et de moelle épinière sans isolement préalable des neurones afin de prendre en compte un maximum d'évènements d'épissages alternatifs liés au développement de la pathologie.
- à partir de sang total périphérique ou de fractions cellulaires sanguines

Les séquences identifiées par DATAS correspondent à des introns et/ou à des exons dont les expressions différentielles par épissage entre les situations pathologiques et la situation saine sont validées par PCR.
La comparaison de ces séquences avec les banques de données permet de classifier les informations obtenues et de proposer une sélection raisonnée des deux séquences dont l'étude est à poursuivre.

### Caractérisation ultérieure des séquences obtenues :

Les séquences validées par PCR peuvent être recherchées dans des modèles complémentaires impliquant des processus de neurodégénération. Ainsi des ARN provenant d'un modèle d'ischémie cérébrale ou des ARN d'un modèle animal de maladie à prions peuvent être utilement étudiés afin de valider l'expression sélective de marqueurs d'ALS ou plus généralement de marqueurs de maladies neurodégénératives.

L'expression des formes d'épissage identifiées sera recherchée dans des échantillons humains représentatifs de différentes pathologies à composantes neurodégénératives :
- prélèvements sanguins de patients atteints de maladies neurodégénératives comme la maladie d'Alzheimer, le Parkinson etc..

### Exemple de cancer : cancer des voies aériennes et digestives supérieures.

La transmission de transgènes à fait l'objet de nombreuses applications expérimentales dans le domaine de la cancérologie expérimentale. Ainsi utilise-ton les allèles dominants de certains gènes cancérogènes pour obtenir des souris transgéniques, alors modèle expérimental pour l'étude du cancer.
Les cancers constituent un groupe hétérogène de maladies caractérisées chacune par un ensemble complexe d'altérations génétiques ayant pour conséquence une prolifération cellulaire anarchique et la dissémination de métastases. Si l'identification des altérations génétiques qui provoquent l'apparition et la progression des cancers apparaît aujourd'hui essentielle pour diagnostiquer et suivre l'évolution tumorale, c'est la perspective d'élaborer de nouveaux diagnostics plus précoces sur la base de cette connaissance qui rend compte de l'importance des enjeux de ces recherches pour l'industrie pharmaceutique. Les gènes dont les altérations peuvent conférer à une cellule des propriétés cancéreuses, sont nombreux. Ils jouent des rôles essentiels non seulement au cours du développement mais aussi tout au long de la vie cellulaire. Ils interviennent ainsi pour assurer des fonctions aussi essentielles que la prolifération, la différenciation, la réparation de l'ADN ou la survie cellulaire. Ceux dont les altérations conduisent à la production de protéines qui activent anormalement le cycle cellulaire sont appelés des oncogènes. On trouve par exemple dans cette catégorie les gènes cellulaires myc et ras. Les anti-oncogènes ont au contraire pour fonction normale de freiner le cycle cellulaire. L'inhibition de leur activité met la cellule sous la seule dépendance des gènes à effet proliférateur et favorise donc la progression tumorale. On trouve dans cette catégorie les gènes RB (rétinoblastome) et P53. A coté des oncogènes et anti-oncogènes, les gènes modulant la mort cellulaire programmée ou apoptose apparaissent comme des acteurs importants de la cancérogenèse. Assimilable à un processus de différenciation cellulaire physiologique, la mort cellulaire programmée est contrôlée génétiquement. Le fait qu'une cellule ait perdu la capacité d'enclencher cette différenciation terminale qui en permet l'élimination, la met en effet en situation de survie anormale et peut favoriser l'émergence d'un clone cellulaire transformé. C'est ce que l'on observe dans les lymphomes folliculaires humains où le gène bcl-2 se trouve sur exprimé en raison de la translocation survenue entre les chromosomes 14 et 18. Cette sur-expression d'un gène à activité anti-apoptotique favorise la survie anormalement longue de populations cellulaires au sein desquels peuvent alors s'accumuler d'autres mutations transformantes. La mort cellulaire programmée ou apoptose est aujourd'hui reconnue comme un mécanisme essentiel à l'élimination de cellules devenues indésirables qu'il s'agisse de cellules infectées par un virus comme de cellules ayant accumulés des mutations les rendant non fonctionnelles ou anormalement proliférantes. Le niveau de complexicité qui régit l'homéostasie cellulaire résulte non seulement du nombre important des acteurs impliqués mais aussi des rôles variés que chacun d'eux peut alternativement jouer en fonction du type cellulaire ou des conditions. L'anti-oncogène p53 ou le proto-oncogène cMyc peuvent par exemple jouer aussi un rôle important dans le contrôle de l'apoptose. Une telle complexité rend nécessaire la mise en oeuvre d'approches assez globales pour analyser des modulations d'expression d'ensembles de gènes et suffisamment spécifiques pour, le plus rapidement possible identifier les altérations les plus pertinentes en regard du diagnostic, du suivi de la progression tumorale ou de l'identification de nouvelles cibles d'actions thérapeutiques. En utilisant différents systèmes d'adressage (régions promotrices) il est possible d'obtenir d'une manière préférentielle l'expression du transgène dans un tissu particulier. On peut ainsi disposer de modèles de tumeurs se développant dans un environnement tissulaire spécifique. Ces différents modèles de tumeurs ciblées laissent entrevoir la possibilité de détecter des signatures spécifiques au niveau des cellules circulantes en fonction de la localisation de la tumeur.

### Modèle de tumeur ciblée sur le foie.

Il existe chez la souris un modèle d'hépatocarcinome (HCC) lié à l'expression restreinte dans le foie des séquences précoces du virus SV40, codant pour les antigènes grand T et petit t (Dubois, N., Bennoun, M., Allemand, I., Molina. T., Grimber, G., Daudet-Monsac, M., Abelanet, R., and Briand, P. (1991) Time course development of differentiated hepatocarcinoma and lung metastasis in transgenic mice. J. Hepatol., 13, 227-239). Le transgène est sous le contrôle du promoteur du gène humain de l'antithrombine III ce qui entraîne une expression précoce et constante des antigènes viraux. De ce fait, la prolifération hépatocellulaire subit une perturbation en deux temps. L'indice de prolifération des hépatocytes transgéniques est proportionnellement supérieure à la normale pendant le développement hépatique (de la naissance à la 5^{ème} semaine), puis diminue sensiblement sans pour autant atteindre l'indice faible caractéristique de l'état quiescent d'un foie normal. Ces souris transgéniques développent de façon systématique des HCC différenciés conduisant à la mort de tous les animaux avant 7 mois. Malgré une dérégulation précoce de la prolifération des hépatocytes, l'hépatomégalie n'apparaît que tardivement. L'analyse des étapes prénéoplasiques précédant l'apparition des HCC a permis de mettre en évidence un mécanisme compensateur par apoptose, maintenant une masse hépatique normale dans ce modèle (Allemand *et al*., 1995). Il est intéressant de noter que cette apoptose s'arrête au moment même où le foie normal entre dans un état quiescent. Au delà de ce point, il semble que l'homéostasie hépatique ne soit plus contrôlée. Une étude systématique de la sensibilité à l'apoptose a révélé que les hépatocytes dérivés de ce modèle transgénique avaient acquis un caractère résistant à la mort cellulaire dépendante du système CD95/Fas (Rouquet N, Allemand I, Molina T, Bennoun M, Briand P and Joulin V. (1995) Fas-dependent apoptosis is impaired by SV40 T-antigen in transgenic liver. Oncogene, 11, 1061-1067

Rouquet N, Allemand I, Grimber G, Molina T, Briand P and Joulin V. (1996) Protection of hepatocytes from Fas-mediated apoptosis by a non-transforming SV40 T-antigen mutant, cell Death & Diff., 3, 91-96) par un mécanisme indépendant d'un épissage alternatif du récepteur CD95/Fas. Cependant, seule une analyse globale des modifications d'épissage peut rendre compte d'une altération de ce processus pour l'ensemble des partenaires moléculaires intervenant dans la voie de signalisation du récepteur CD95/Fas. Ce modèle transgénique représente un outil idéal pour identifier 1) les modifications de l'expression génique accompagnant la transition prénéoplasie vers néoplasie, que ce soient des gènes indispensables à la transformation (oncogènes) ou des gènes s'opposant à la progression tumorale (gènes apoptotiques) 2) des signatures circulantes du développement du cancer liées à l'échappement à partir de la tumeur de cellules tumorales 3) d'évènements d'altération de l'expression génique dans les cellules du sang caractéristiques du développement du cancer.

### Identification des signatures spécifiques

L'approche différentielle qualitative est menée sur des ARN extraits de foie et de cellules du sang de souris normales et de souris développant des hépatocarcinomes (HCC) liés à l'expression de l'antigène T de SV40 sous contrôle du promoteur de l'AntiThrombine III. Les animaux contrôles et les animaux exprimant le transgène sont choisis à différents âges permettant d'avoir accès aux stades très précoces, prénéoplasiques et néoplasiques caractérisés notamment dans ce modèle par une activation puis une inactivation de l'apoptose nécessaire à l'homéostase hépatique.

### Utilisation des séquences identifiées

Les modulations de l'expression de ces séquences pourront être ensuite recherchées dans des biopsies de tumeurs humaines afin d'en élargir le champ d'application, en thérapie humaine et en diagnostic.

Ces ADNc sont utilisés pour suivre l'évolution tumorale dans ce modèle transgénique et dans une série de modèles d'HCC murins produits par transgenèse (Bennoun M, Grimber G, Couton D, Seye A, Molina T, Briand P and Joulin V. (1998) The amino-terminal région of SV40 large T antigen is sufficient to induce hepatic tumours in mice Oncogene, 17, *sous presse*). Ainsi en utilisant les ADNc spécifiques détectés à différents stades très précoces dans les cellules sanguines, avant l'apparition des tumeurs, on peut prédire dans une population mixte de souris saines et de souris transgèniques, celles qui vont développer un cancer.

Des sondes nucléotidiques ou des amorces de PCR dérivés de ces ADNc spécifiques des tumeurs peuvent être utilisées pour rechercher l'expression, dans des biopsies de tumeurs humaines, des formes d'épissage identifiées et/ ou les ARN dont la quantité est altérée dans ce modèle.

De même, les sondes identifiées dans le sang des animaux à différents stades du développement tumoral peuvent être également utilisées afin de détecter des signatures communes avec des prélèvements de sang de patients atteints de cancers.

Dans une stratégie qui utilise des banques d'ADNc obtenues selon les procédés de l'invention décrits plus haut, une sonde totale préparée à partir de prélèvement sanguins de patients atteints de cancer, peut également être utilisée pour la recherche de signatures communes aux différentes banques issues de modèles murins à différents stades du développement tumoral d'une part et à partie de biopsies de différents types tumoraux humains d'autre part. Ces hybridations sont réalisées selon les techniques connues de l'homme de métier (voir notamment les conditions d'hybridation décrites dans la demande WO 99/46403.

### RECHERCHE DU DIAGNOSTIC PREDICTIF CHEZ L'HOMME

La méthodologie décrite dans ce chapitre pourra être mise en oeuvre indifféremment en utilisant les techniques d'analyse quantitative et qualitative décrites ci-avant. L'invention privilégie néanmoins l'utilisation et la recherche de marqueurs liés aux altérations qualitatives de l'expression des gènes en raison des avantages décrits précédemment.

L'invention décrit l'identification et la constitution de banques de signatures caractéristiques de l'évolution d'une pathologie à partir de biopsies. Ainsi il est possible d'établir des banques de séquences d'ADNc représentatifs de l'évolution et de la localisation de ces pathologies. Il est donc possible de rechercher, à partir d'un échantillon sanguin, la présence de signatures ADNc identiques à celles présentes dans les banques. La présence de signatures communes souligne alors la présence d'acides nucléiques présentant les mêmes altérations que celles présentes dans les biopsies des pathologies suspectées, très vraisemblablement issues de cellules issues de tissus pathologiques.

La mise en oeuvre de cette recherche est notamment basée sur la confection de sondes à partir de cellules sanguines et l'hybridation de ces sondes avec des filtres qui regroupent différents marqueurs spécifiques de telle ou telle pathologie.

L'invention décrit la possibilité d'identifier des altérations de l'expression des gènes à partir de cellules sanguines et ceci à partir de modèles expérimentaux mimant tout ou partie d'une pathologie humaine (exemple modèle de souris ALS ou modèle de tumeur hépatique).
L'utilisation de sondes nucléiques dérivées de cellules sanguines de patients atteints ou non de la pathologie ciblée (maladie neurodégénérative, cancer etc..) permet de rechercher l'existence de signatures communes avec les banques prédictives expérimentales créées à partir des modèles pathologiques expérimentaux. L'apparition de signatures communes constitue un diagnostic d'un risque de développement d'une telle pathologie chez l'individu soumis au diagnostic.

L'invention permet également de procéder de la manière suivante :
- Des prélèvements sanguins de patients atteints ou non d'une pathologie ciblée sont mélangés afin de constituer un stock d'ARN représentatif des états pathologique et sain.
- Ces ARNs sont soumis à des analyses différentielles selon les techniques décrites dans l'invention et des banques d'ADNc caractéristiques des états pathologiques et sains sont constituées.
- Ces banques d'ADNc sont ensuite validées par hybridation à l'aide de sondes préparées à partir d'échantillons individuels de sang de patients ou de sujets sains.
- Les banques ainsi validées sont ensuite analysées par l'utilisation de sondes préparées à partir de prélèvements de sang d'une large population de sujets consultant un médecin pour des examens de routine. Ces banques constituent un outil diagnostic propre à l'invention. Ainsi un patient effectuant une mammographie pour dépistage d'un cancer du sein, peut être prélevé d'un petit échantillon de sang. Une sonde nucléique préparée à partir d'un tel échantillon permet ensuite de rechercher très précocement des signes probables de l'évolution d'un cancer même en l'absence d'image à la radiographie.

L'invention peut être utilisée sous forme de BioPuce. La BioPuce exploite les propriétés de la réaction d'hybridation par laquelle deux brins d'ADN dits complémentaires se lient l'un à l'autre d'une manière très spécifique. L'invention peut être utilisée en recherchant spécifiquement un ou plusieurs marqueurs ADN de la pathologie en utilisant une technique d'amplication de l'ADN à l'aide d'amorces oligonucléotidiques spécifiques de l'ADN à rechercher.

## Revendications

1. Procédé de détection à distance de la présence d'une pathologie chez un sujet, comprenant la détermination in vitro, dans un échantillon de cellules sanguines du sujet, de la présence de forme(s) d'épissages alternatifs de gène(s) caractéristique(s) de la pathologie, les cellules sanguines de l'échantillon étant distinctes des tissus pathologiques.

2. Procédé selon la revendication 1, **caractérisé en ce qu'**il comprend (i) la préparation d'acides nucléiques de l'échantillon et (ii) l'hybridation des acides nucléique préparés avec au moins une banque d'acides nucléiques caractéristiques de forme(s) d'épissage de gène(s), le profil d'hybridation indiquant la présence de la pathologie.

3. Procédé selon la revendication 2, **caractérisé en ce que** les acides nucléiques de la banque sont préparés par un procédé comprenant (i) l'obtention d'une première préparation d'acides nucléiques à partir de cellules sanguines d'un organisme présentant la pathologie, (ii) l'obtention d'une préparation d'acides nucléiques de référence à partir de cellules sanguines d'un organisme ne présentant pas ladite pathologie, (iii) une étape d'hybridation entre ladite première préparation et la préparation de référence et la récupération, à partir des hybrides formés, des acides nucléiques correspondant à des épissages différentiels.

4. Procédé selon la revendication 2 ou 3, **caractérisé en ce que** la banque comprend de 50 à 5000 acides nucléiques.

5. Procédé selon l'une des revendications 1 à 4, **caractérisé en ce que** la ou les banques sont déposées sur un support.

6. Procédé selon l'une des revendications 2 à 5, **caractérisé en ce que** les acides nucléiques préparés à partir de l'échantillon sont des ARN totaux ou messagers, ou des ADNc dérivés de ceux-ci.

7. Procédé selon la revendication 6, **caractérisé en ce que** les acides nucléiques sont marqués.

8. Procédé selon l'une quelconque des revendications précédentes, **caractérisé en ce que** les cellules sanguines sont des cellules nucléées.

9. Procédé selon la revendication 8, **caractérisé en ce que** les cellules sanguines nucléées comprennent des lymphocytes, des macrophages, des monocytes et/ou des cellules dendritiques.

10. Procédé selon l'une des revendications précédentes, pour la détection in vitro du stade d'évolution d'une pathologie chez un sujet.

11. Procédé selon l'une des revendications précédentes, pour la détection in vitro de la localisation d'une pathologie chez un sujet.

12. Procédé selon l'une quelconque des revendications précédentes pour la détection in vitro d'une pathologie liée au vieillissement ou impliquant une prolifération cellulaire anormale.

13. Kit utilisable pour la mise en oeuvre d'un procédé selon l'une quelconque des revendications précédentes comprenant, ordonnés sur un support solide adapté à l'étalement d'acides nucléiques, 50 à 5000 clones d'acides nucléiques comprenant des séquences spécifiques d'exons ou d'introns de gènes épissés façon différentielle entre des cellules sanguines d'un organisme en situation pathologique et des cellules sanguines d'un organisme en situation saine.

## Patentansprüche

1. Verfahren zum indirekten Nachweis des Vorliegens einer Krankheit bei einer Person, umfassend die in vitro Bestimmung des Vorliegens einer Form oder Formen alternativer Spleißungen eines für die Krankheit charakteristischen Gens oder Gene in einer Probe von Blutzellen der Person, wobei die Blutzellen der Probe sich von pathologischen Geweben unterscheiden.

2. Verfahren nach Anspruch 1, **dadurch gekennzeichnet, dass** es umfasst (i) die Präparation von Nukleinsäuren der Probe und (ii) die Hybridisierung der präparierten Nukleinsäuren mit wenigstens einer Bank von Nukleinsäuren, die für eine Spleißform oder Spleißformen eines Gens oder Gene charakteristisch sind, wobei das Hybridisierungsmuster das Vorliegen der Krankheit anzeigt.

3. Verfahren nach Anspruch 2, **dadurch gekennzeichnet, dass** die Nukleinsäuren der Bank mit einem Verfahren präpariert sind, umfassend (i) die Gewinnung eines ersten Präparates von Nukleinsäuren aus Blutzellen eines die Krankheit aufweisenden Organismus, (ii) die Gewinnung eines Präparates von Referenz-Nukleinsäuren aus Blutzellen eines die besagte Krankheit nicht aufweisenden Organismus, (iii) einen Hybridisierungsschritt zwischen besagtem ersten Präparat und dem Referenz-Präparat und aus den gebildeten Hybriden die Wiedergewinnung der Nukleinsäuren, die differentiellen Spleißungen entsprechen.

4. Verfahren nach Anspruch 2 oder 3, **dadurch gekennzeichnet, dass** die Bank 50 bis 5000 Nukleinsäuren umfasst.

5. Verfahren nach einem der Ansprüche 1 bis 4, **dadurch gekennzeichnet, dass** die Bank oder Banken auf einem Träger angelagert sind.

6. Verfahren nach einem der Ansprüche 2 bis 5, **dadurch gekennzeichnet, dass** die aus der Probe präparierten Nukleinsäuren Gesamt-RNA oder Messenger-RNA oder von diesen abstammende cDNA sind.

7. Verfahren nach Anspruch 6, **dadurch gekennzeichnet, dass** die Nukleinsäuren markiert sind.

8. Verfahren nach einem der vorhergehenden Ansprüche, **dadurch gekennzeichnet, dass** die Blutzellen kernhaltige Zellen sind.

9. Verfahren nach Anspruch 8, **dadurch gekennzeichnet, dass** die kernhaltigen Blutzellen Lymphozyten, Makrophagen, Monozyten und/oder dendritische Zellen umfassen.

10. Verfahren nach einem der vorhergehenden Ansprüche zum in vitro Nachweis des Entwicklungsstadiums einer Krankheit bei einer Person.

11. Verfahren nach einem der vorhergehenden Ansprüche zum in vitro Nachweis der Lokalisation einer Krankheit bei einer Person.

12. Verfahren nach einem der vorhergehenden Ansprüche zum in vitro Nachweis einer Krankheit, die mit der Alterung verbunden ist oder eine anormale Zellproliferation umfasst.

13. Kit, das zur Durchführung eines Verfahrens nach einem der vorhergehenden Ansprüche verwendet sein kann, umfassend 50 bis 5000 Nukleinsäure-Klone, die auf einem für das Ausbreiten von Nukleinsäuren geeigneten festen Träger angeordnet sind und Sequenzen umfassen, die für Exons und Introns von Genen spezifisch sind, die zwischen Blutzellen eines Organismus in einer pathologischen Situation und Blutzellen eines Organismus in einer gesunden Situation in differentieller Weise gespleißt sind.

## Claims

1. A process for the remote detection of the presence of a pathology in a subject, comprising determining, in vitro, in a sample of blood cells from the subject, the presence of alternative splicing form(s) of gene(s) characteristic of the pathology, the blood cells from the sample being distinct from the pathological tissues.

2. The process of claim 1, **characterized in that** it comprises (i) preparing nucleic acids from the sample and (ii) hybridizing the nucleic acids so prepared with at least one nucleic acid library characteristic of splicing form(s) of gene(s), the hybridization profile indicating the presence of the pathology.

3. The process of claim 2, **characterized in that** the nucleic acids of the library are prepared by a process comprising (i) obtaining a first nucleic acid preparation from blood cells of an organism presenting the pathology, (ii) obtaining a reference nucleic acid preparation from blood cells of an organism not presenting said pathology, (iii) a hybridization step between said first preparation and said reference preparation, and recovering, from the hybrids formed, the nucleic acid corresponding to differential splicings.

4. The process of claim 2 or 3, **characterized in that** the library comprises from 50 to 5000 nucleic acids.

5. The process according to any one of claims 1 to 4, **characterized in that** the library is deposited on a support.

6. The process according to any one of claims 2 to 5, **characterized in that** the nucleic acids prepared from the sample are total or messenger RNAs, or cDNAs derived therefrom.

7. The process according to claim 6, **characterized in that** the nucleic acids are labelled.

8. The process according to any one of the preceding claims, **characterized in that** the blood cells are nucleated cells.

9. The process according to claim 8, **characterized in that** the nucleated blood cells comprise lymphocytes, macrophages, monocytes and/or dendritic cells.

10. The process according to any one of the preceding claims, for the in vitro detection of the stage of progression of a pathology in a subject.

11. The process according to any one of the preceding claims, for the in vitro detection of the site of a pathology in a subject.

12. Process according to any one of the preceding claims, for the in vitro detection of a pathology related to ageing or involving an abnormal cell proliferation.

13. A kit useful for implementing a process according to any one of the preceding claims comprising, ordered on a support solid suitable for the spotting of nucleic acids, 50 to 5000 nucleic acid clones comprising sequences specific for exons or introns of genes that are differentially spliced between blood cells of an organism in a pathological condition and blood cells from an organism in healthy condition.
